# EUROPEAN PATENT APPLICATION

(11) **EP 1 112 725 A1**
(43) Date of publication of application: **04.07.2001**
(21) Application number: 99933227.3
(22) Date of filing: 03.08.1999
(51) Int. Cl.: A61F 2/06

(54) **ARTIFICIAL BLOOD VESSEL**

(30) Priority: 07.09.1998 JP 25208798
(71) Applicant: Tapic International Co., Ltd., Tokyo 105-0001 (JP); Shimizu, Yasuhiko, Uji-shi, Kyoto 611-0002 (JP)
(72) Inventor: SHIMIZU, Yasuhiko, Kyoto 611-0002 (JP); TAMURA, Nobushige, Kyoto-shi, Kyoto 603-8023 (JP); TERAI, Hiromu, Kyoto-shi, Kyoto 607-8072 (JP); NAKAMURA, Tatsuo, Cosmo-today 1109, Kamigyo-ku, Kyoto-shi, Kyoto 602-8342 (JP); YAMAMOTO, Yasumichi, Kyoto-shi, Kyoto 606-8156 (JP)
(74) Representative: Stuart, Ian Alexander
(86) International application number: JP9904157
(87) International publication number: WO0013612

(57) **Abstract**

An artificial blood vessel having the following characteristics: (1) having antithrombotic properties; (2) having tissue affinity; (3) enabling endothelium formation to occur quickly; and (4) allowing regeneration of host cells, and its production method. The artificial blood vessel is one extracted from a living body of a heterogeneous species from which all of the cellular components of the blood vessel has been removed. The production method of the artificial blood vessel comprises a step in which a blood vessel extracted from a living body of a heterogeneous species is washed with surfactant to remove all cellular components of said blood vessel.

## Description

### Technical Field

The present invention relates to an artificial blood vessel comprised of a material originating from a living body, and its production method, and more particularly, to an artificial blood vessel using a blood vessel extracted from a living body of a heterogeneous species as its base material, and its production method.

### Background Art

The United States Society of Vascular Surgery indicated the following requirements as conditions to be satisfied by artificial blood vessels (1954).
(1) Physical properties must remain stable in the body.
(2) Must be chemically stable and non-toxic.
(3) Must not cause a foreign body reaction.
(4) Must not be carcinogenic.
(5) Must not cause an allergic reaction.
(6) Must have large fatigue strength.
(7) Must be able to be produced easily and at low cost.
(8) Must be able to be easily disinfected.

More recently, in addition to these safety in the material, artificial blood vessels are required to have both biocompatibility and performance after implantation as indicated below.
(9) Must have excellent antithrombotic properties.
(10) Must have compliance comparable to natural blood vessels.
(11) Must allow the obtaining of rapid and satisfactory healing of new tunica intima.

On the other hand, some major examples of artificial blood vessels that are currently used or have been reported include those indicated below.
A. Those having Dacron (trade name) as their base material
B. Those having ePTFE as their base material
C. Those having human umbilical cord as their base material
D. Those having bovine internal thoracic artery as their base material

However, in the case of those having Dacron as their base material, cases have been reported in which aneurysms have formed during long-term use. Moreover, covering by endothelial cells has also not been observed in clinical cases. More recently, although artificial blood vessels in which said base material is coated with collagen or gelatin have been used as large-aperture artificial blood vessels, the occurrence of post-surgical fever of unknown origin, thought to be caused by an endotoxin present at the stage of production, has been sporadically observed.

In the case of those having ePTFE as their base material, regeneration of endothelial cells is not performed due to the low level of tissue affinity, and satisfactory results have yet to be obtained with respect to long-term patency in small-aperture artificial blood vessels due to, for example, the formation of pannus at anastomosed sections.

In the case of those using human umbilical cord as their base material, in addition to susceptibility to the occurrence of ruptures at anastomosed sections and defective regeneration of endothelial cells resulting in a poor patency ratio, the material is difficult to acquire, and the issue of purion, which have recently presented problems, has yet to be overcome.

In the case of those using bovine internal thoracic artery as their base material, although this is the only artificial blood vessel that uses a material originating from a living body of a heterogeneous species, since the bovine cells remain, in order to avoid a rejection reaction caused by heterogeneous tissue and heterogeneous cells, said residual cells must be completely crosslinked and fixed with reagents. Thus, since the artificial artery is not replaced whatsoever by the host's own body, there is no promotion of endothelium formation, and the host's cells also do not regenerate. Moreover, these artificial blood vessels are currently not used due to their poor patency ratio.

As has been described above, none of the artificial blood vessels of the prior art are able to satisfy the previously mentioned conditions, and in the case of small-aperture blood vessels of 6 mm or less in particular, an artificial blood vessel that has long-term patency able to withstand clinical applications has yet to be obtained.

### Disclosure of the Invention

The object of the present invention is to provide an artificial blood vessel and its production method that is able to solve the above-mentioned problems of artificial blood vessels of the prior art. In particular, the object of the present invention is to provide an artificial blood vessel that:
(1) has antithrombotic properties;
(2) has tissue affinity;
(3) enables endothelium formation to occur quickly; and,
(4) allows regeneration of host cells,
and its production method.

The present invention is a blood vessel extracted from a living body of a heterogeneous species comprising removing all of the cellular components of said blood vessel.

In addition, the present invention is a production method of the above artificial blood vessel that comprises a step in which a blood vessel extracted from a living body of a heterogeneous species is washed with surfactant to remove all cellular components of said blood vessel.

### Best Mode for Carrying Out the Invention

In the artificial blood vessel of the present invention, a "living body of a heterogeneous species" refers to an animal other than a human such as a cow, pig, dog or monkey, while "removing all of the cellular components" refers to the absence of cellular components with HE stain or being IgG stain negative. Moreover, it is preferable that the artificial blood vessel of the present invention also be negative for MHC antigen stain (even though it may be falsely positive, in the case there are no cellular components in the form of heterogeneous antigens in microscopic findings, it is treated as being negative).

Furthermore, the artificial blood vessel of the present invention may apply a substance having antithrombotic properties to wall surface of its lumen, examples of which include heparin, argatroban and plasmin.

Here, although there are no particular restrictions on the extracting site of the blood vessel used as the base material, as a general rule, it is preferable that the aperture and constituent components be equivalent to the blood vessels of the patient in which the artificial blood vessel is to be applied, and the site preferably has physical properties, and particularly strength, that are equivalent to the blood vessels of said patient.

The surfactant used to wash said extracted blood vessel is a non-ionic surfactant, and is preferably a polyoxyethylene derivative represented by, for example, "Triton X-100", "Lubrol PX" or the "Tween series".

In addition, washing is specifically performed by immersing for 12-72 hours in a 0.1-3 wt% surfactant solution. If the concentration of the surfactant is less than 0.1 wt%, removal of the cellular components of the washed blood vessel is inadequate, while if the concentration of surfactant exceeds 3 wt%, the surfactant remains in the washed blood vessel. On the other hand, if the immersion time is less than 12 hours, removal of the cellular components of the washed blood vessel is inadequate, and if the immersion time exceeds 72 hours, extracellular components that are to remain in the washed blood vessel (at least collagen and elastic fibers must remain) become damaged.

In this washing procedure, it is preferable that the surfactant solution be replaced with a fresh solution at predetermined intervals. This is done to maintain washing capacity. Furthermore, washing with distilled water when replacing the surfactant solution is even more desirable. This is because washing effects are enhanced since the cellular components to be removed undergo swelling and rupture.

In addition, ultrasonic washing may be additionally performed before and/or after washing by surfactant solution. This enables cellular components to be more completely removed from the base material.

Here, not only the washing procedure using surfactant solution (including washing by distilled water), but also the ultrasonic washing procedure and other necessary procedures (when used) are performed aseptically. This is done to eliminate the possibility of contamination by foreign substances (including not only those have a physical effect, but also those that have a biochemical effect) from the outside.

Furthermore, in the case of applying a substance such as heparin that has antithrombotic properties to the wall surface of the lumen of the artificial blood vessel of the present invention, the method of applying said substance having antithrombotic properties should be applied in accordance with routine methods. As an example of the case of using heparin as said substance having antithrombotic properties, a method may be employed in which a base material on which a washing procedure using surfactant has been performed is immersed for 2-72 hours in a 0.1-5 wt% heparin solution. Here, if the heparin concentration is less than 0.1 wt%, the amount of heparin binding is inadequate (which results in heparin rapidly disappearing from the blood vessel lumen and preventing obtaining of the desired antithrombotic properties), while if the heparin concentration exceeds 5 wt%, the amount of heparin binding becomes excessive (which results heparin remaining for a long time in the blood vessel lumen and inhibiting the regeneration of endothelial cells), thereby making this undesirable. On the other hand, if the immersion time is less than 2 hours, the amount of heparin binding is inadequate, while if the immersion time exceeds 72 hours, the amount of heparin binding becomes excessive, thereby making this undesirable as well.

Here, prior to the application of heparin, glutaraldehyde (to be abbreviated as GA) is applied to the base material on which the above washing by surfactant has been completed to crosslink and bond protamine to the lumen surface of said base material. This is because said protamine and heparin undergo ionic bonding in the next heparin solution immersion procedure, as a result of which heparin is applied to said base material. Furthermore, the objective of application of GA to said base material is only crosslinking and bonding of protamine, and must not be performed to an extent that impairs replacement of the artificial blood vessel to host cells.

Furthermore, a method involving the use of hydrophilic polyepoxy compound ("Deacol" EX-512: Nakase Kasei Kogyo) may be used without using GA for the method of applying heparin.

The following provides a more detailed explanation of the present invention based on one of its embodiments.

Fresh carotid artery extracted from a pig (to be simply referred to as the "base material") was washed with surfactant solution (Sigma, "Triton X-100", concentration: 1 wt%). Washing was performed at 38°C for 48 hours. Furthermore, the surfactant solution was replaced every 12 hours. Washing with distilled water was performed for about 1 hour when the surfactant solution was replaced.

After washing the base material on which said washing with surfactant solution was completed with distilled water (at 38°C for 48 hours), the lumen of said base material was filled with protamine solution (concentration: 1 wt%), after which the base material was immersed (for 5-10 minutes) in GA solution (concentration: 1 wt%) while applying pressure (80-100 mmHg) to the lumen of said base material.

After washing said GA-treated base material with physiological saline (at 38°C for 30 minutes), the base material was immersed (at 40°C for 48 hours) in heparin solution (concentration: 1 wt%) and then removed and stored in a cool, dark location.

Furthermore, the entirety of the above procedure was performed aseptically.

Although the wall thickness was somewhat thin, the resulting artificial blood vessel easily handled in the same manner as the host's artery. When observed microscopically, the cells had completely disappeared, and only collagen and elastic fibers were observed. Furthermore, there were no cellular components observed in HE staining, and the artificial blood vessel was negative for both anti-pig IgG staining and MHC antigen staining.

The usefulness of the artificial blood vessel produced in the above manner was verified according to the procedure described below.
(1) Test animals: Beagle dogs (5)
(2) Ketalar (trade name: Sankyo), Celactal (trade name: Bayer) and atropine sulfate were administered by intramuscular injection to the test animals together with applying endotracheal intubation. During surgery, anesthesia was maintained with halothane.
(3) After intravenously administering 1 ml of heparin, the abdominal aorta of the test animals was blocked and a 5-10 mm section was resected. The above artificial blood vessel (outer diameter: 7 mm, length: 20 mm) was anastomosed at that site (dorsal side: 7-0 polypropylene suture, and abdominal side: Auto suture "VCS size M", were respectively used for anastomosis).
(4) The presence or absence of constriction was confirmed by angiography 20 days later. (There was no constriction observed in any of the test animals.)
(5) The artificial blood vessels were extracted evaluated after 3 weeks in two of the test animals and after 18 weeks in three of the test animals.

The results were as shown below.

The patency ratio was 100%. Macroscopically, although the central portion of the artificial blood vessel was partially not covered by endothelium after 3 weeks, endothelium growth was observed from the anastomosed portions. After 18 weeks, endothelial cells were confirmed to have regenerated in all test animals.

Microscopic findings were as described below.

### (1) After 3 weeks:

Endothelial cells were regenerating near the anastomosed portions, and smooth muscle cells were observed in the tunica intima. Although the internal elastic plate was also regenerating, complete consistency was not observed. Invasion by neutrophils and plasma cells was observed on the outside, and partially observed in the tunica intima as well. Deposition of fibrin was observed at those sites of the central portion of the artificial blood vessel where endothelial cells were not present.

### (2) After 18 weeks:

Endothelial cells had completely regenerated and there were no sites where they were missing. Although phlogocytes (inflammatory cells) were observed in some portions, they were extremely mild. Host connective tissue cells, including macrophages and fibroblasts, were observed both inside the lumen and on the outside of the artificial blood vessels, indicating that the artificial blood vessel was gradually being absorbed. The internal elastic plate had regenerated more distinctly as compared with after 3 weeks, and continuity was observed.

### Industrial Utilization

Although long-term patency is the most important element required of artificial blood vessels, as the aperture of the vessel becomes smaller, this becomes more difficult to achieve. In addition, the use of a heterogeneous living body as the supply source of the base material has also considered to present problems with respect to its antigenicity.

However, in the artificial blood vessel of the present invention, since cellular components are removed from said base material by a washing procedure using surfactant, resulting in the use of an acellular matrix containing only fiber components, antigenicity is extremely low enabling this artificial blood vessel to significantly contribute to the progress of industry with respect to having solved the problem associated with supplying base materials. In addition, since aggressive steps have been taken to acquire antithrombotic properties in order to enhance long-term patency of the artificial blood vessel, a superior artificial blood vessel is able to be provided.

Moreover, since said acellular matrix of the artificial blood vessel of the present invention is absorbed and replaced by host cells while promoting regeneration of said host cells, the host's own vessel can be completely regenerated and restored, thereby making it possible to provide an extremely useful artificial blood vessel.

## Claims

1. An artificial blood vessel comprising removing all of the cellular components of a blood vessel extracted from a living body of a heterogeneous species.

2. A production method of the artificial blood vessel according to claim 1 that comprises a step in which a blood vessel extracted from a living body of a heterogeneous species is washed with surfactant to remove all cellular components of said blood vessel.
